(19) Europäisches Patentamt
European Patent Office
Office européen des brevets

(11) EP 0 501 268 B1

(12) **EUROPÄISCHE PATENTSCHRIFT**

(45) Veröffentlichungstag und Bekanntmachung des Hinweises auf die Patenterteilung:
**12.06.1996 Patentblatt 1996/24**

(21) Anmeldenummer: **92102588.8**

(22) Anmeldetag: **17.02.1992**

(51) Int. Cl.$^6$: **C07C 25/18**, C09K 19/12, C09K 19/34, C09K 19/30, C07D 239/26, C07D 319/06, C07D 213/26, G02F 1/13

(54) **Flüssigkristalline Verbindungen mit endständigem 1-Alkinylrest**
Liquid crystal compounds ending with a 1-alkinyl group
Composés cristaux liquides avec un groupe alkinyl-1 en bout de chaîne

(84) Benannte Vertragsstaaten:
**CH DE FR GB IT LI NL**

(30) Priorität: **25.02.1991 CH 557/91**

(43) Veröffentlichungstag der Anmeldung:
**02.09.1992 Patentblatt 1992/36**

(73) Patentinhaber: **F. HOFFMANN-LA ROCHE AG**
**CH-4002 Basel (CH)**

(72) Erfinder:
- **Buchecker, Richard**
  **CH-8008 Zürich (CH)**
- **Schadt, Martin**
  **CH-4411 Seltisberg (CH)**

(74) Vertreter: **Cottong, Norbert A. et al**
**F.Hoffmann-La Roche AG**
**Patent Department (PLP),**
**124 Grenzacherstrasse**
**4070 Basel (CH)**

(56) Entgegenhaltungen:
**EP-A- 0 409 634** **EP-A- 0 427 957**
**GB-A- 2 111 992**

- **CHEMICAL ABSTRACTS, vol. 107, no. 4, 27. Juli 1987, Columbus, Ohio, US; abstract no. 31345, Seite 536 ;Spalte 2 ; & JP-A-61 263 955 (DAINIPPON INK AND CHEMICALS, INC.)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 501 268 B1

Printed by Rank Xerox (UK) Business Services
2.12.3/3.4

## Beschreibung

Die vorliegende Erfindung betrifft neue Verbindungen mit endständigem 1-Alkinylrest, deren Herstellung, flüssigkristalline Gemische, die solche Verbindungen enthalten, sowie die Verwendung dieser Verbindungen bzw. Gemische für elektro-optische Zwecke.

Flüssige Kristalle werden vor allem als Dielektrika in Anzeigevorrichtungen verwendet, da die optischen Eigenschaften solcher Substanzen durch eine angelegte Spannung beeinflusst werden können. Elektro-optische Vorrichtungen auf der Basis von Flüssigkristallen sind dem Fachmann bestens bekannt und können auf verschiedenen Effekten beruhen. Derartige Vorrichtungen sind beispielsweise Zellen mit dynamischer Streuung, DAP-Zellen (Deformation aufgerichteter Phasen), Gast/Wirt-Zellen, TN-Zellen mit verdrillt nematischer ("twisted nematic") Struktur, STN-Zellen ("supertwisted nematic"), SBE-Zellen ("super-birefringence effect") und OMI-Zellen ("optical mode interference"). Die gebräuchlichsten Anzeigevorrichtungen beruhen auf dem Schadt-Helfrich-Effekt und besitzen eine verdrillt nematische Struktur.

Die Flüssigkristallmaterialien müssen eine gute chemische, photochemische und thermische Stabilität und eine gute Stabilität gegenüber elektrischen Feldern besitzen. Ferner sollten sie über einen möglichst breiten Bereich eine geeignete Mesophase aufweisen (beispielsweise für die oben genannten Zellen eine nematische bzw. eine cholesterische Phase), aber trotzdem ausreichend niedere Viskosität besitzen und in den Zellen kurze Ansprechzeiten, tiefe Schwellenspannungen und einen hohen Kontrast ermöglichen. Weitere Eigenschaften, wie die elektrische Leitfähigkeit, die dielektrische Anisotropie und die optische Anisotropie, müssen je nach Anwendungsgebiet und Zellentyp unterschiedlichen Anforderungen genügen. Beispielsweise sollten Materialien für die Zellen mit verdrillt nematischer Struktur eine positive dielektrische Anisotropie und eine möglichst geringe elektrische Leitfähigkeit aufweisen. Für DAP-Zellen sind Komponenten mit negativer dielektrischer Anisotropie notwendig.

Um diese zum Teil widersprüchlichen Anforderungen zu erfüllen, müssen in der Regel Mischungen mit bis zu etwa 15 Komponenten hergestellt werden. Es ist daher wichtig, dass die Komponenten untereinander gut mischbar sind und eine ausreichende Löslichkeit aufweisen.

Um ausreichend breite Mesophasenbereiche zu erzielen, müssen den Mischungen meist klärpunkts-erhöhende Komponenten zugesetzt werden, welche jedoch die Viskosität und die elektro-optischen Eigenschaften nachteilig beeinflussen können. Ferner ergeben Materialien mit niedriger optischer Anisotropie, welche z.B. für aktiv adressierte Flüssigkristallanzeigen von Interesse sind, häufig smektische Tendenzen und führen meist auch zu einer Erhöhung der Schwellenspannung und/oder der Ansprechzeiten. Weiterhin besitzen unpolare Materialien mit hoher optischer Anisotropie oft nur smektische Mesophasen oder gar keine flüssigkristallinen Eigenschaften.

Gegenstand der Erfindung sind die Verbindungen der allgemeinen Formel

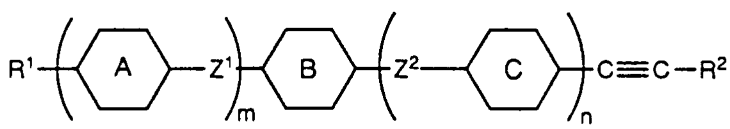 I

worin

die Ringe A und C unabhängig voneinander trans-1,4-Cyclohexylen, 1-4-Phenylen, trans-1,3-Dioxan-2,5-diyl, Pyridin-2,5-diyl oder Pyrimidin-2,5-diyl sind, mit der Massgabe, dass höchstens einer der Ringe A und C Pryidin-2,5-diyl oder Pyrimidin-2,5-diyl sein kann,

der Ring B 2,3-difluorsubstituiertes 1,4-Phenylen ist,

$Z^1$ oder $Z^2$ eine einfache Bindung, Aethylen, Oxymethylen oder Methylenoxy bedeuten,

m und n unabhängig voneinader 0, 1 oder 2 bedeuten, wobei die Summe m + n 1 oder 2 beträgt,

$R^1$ $C_{1-10}$-Alkyl, $C_{2-9}$-Alkoxyalkyl oder $C_{1-9}$-Alkoxy bedeutet, und

$R^2$ $C_{1-10}$-Alkyl bedeutet,

mit der Massgabe, dass zwei 1,4-Phenylenringe nur durch eine einfache Bindung miteinander verknüpft sein können.

Die erfindungsgemässen Verbindungen sind unpolare Verbindungen mit vergleichsweise sehr breiten Mesophasebereichen, hohen Klärpunkten und ausgeprägter nematischer Tendenz. Trotz der hohen Klärpunkte besitzen sie erstaunlich niedere Viskosität und günstige elektro-optische Eigenschaften, insbesondere ermöglichen sie kurze Schaltzeiten, niedrige Schwellenspannungen und je nach Wahl von $R^1$ eine Modifizierung der elastischen Eigenschaften. Ferner kann die optische Anisotropie in einem breiten Bereich variiert werden; beispielsweise besitzen die Verbindungen der Formel I, worin die Ringe A und C trans-1,4-Cyclohexylen und/oder trans-1,3-Dioxan-2,5-diyl bedeuten, niedrige optische Anisotropien und die Verbindungen der Formel I, worin die Ringe A und C 1,4-Phenylen bedeuten, besonders hohe optische Anisotropien.

Aufgrund der ausgezeichneten Eigenschaften bieten die erfindungsgemässen Verbindungen ferner die Möglichkeit, die Zahl der Komponenten im Gemisch zu verringern und damit die Gemische wesentlich zu vereinfachen. Hierbei ist

von Vorteil, dass die erfindungsgemässen Verbindungen mit bekannten Materialien gut mischbar und aufgrund der niedrigen Schmelzenthalpien auch in hohen Konzentrationen gut löslich sind.

Es hat sich herausgestellt, dass die erfindungsgemässen Verbindungen sehr vorteilhaft für DAP-Anwendungen geeignet sind, aber auch für STN-Mischungen einsetzbar sind.

Unter "$C_{1-10}$-Alkyl", "$C_{2-9}$-Alkoxyalkyl" und "$C_{1-9}$-Alkoxy" für $R^1$ und/oder $R^2$ sind sowohl geradkettige als auch verzweigte Gruppen zu verstehen.

Sowohl die Stellung des Stickstoffatoms oder der Stickstoffatome im Pyridin-2,5-diylring bzw. Pyrimidin-2,5-diylring (Ring A oder C) als auch die Stellung der Sauerstoffatome des trans-1,3-Dioxan-2,5-diylrings (Ring A und/oder Ring C) weist nur in die Richtung der Dreifachbindung -C ≡ C-, d.h. ein allfälliger solcher Ring ist in der obigen Formel I wie folgt angeordnet:

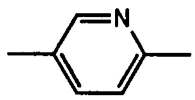 , 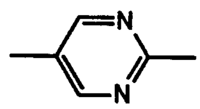 bzw. 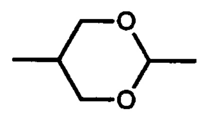

Falls $Z^1$ oder $Z^2$ Oxymethylen ($OCH_2$) oder Methylenoxy ($CH_2O$) bedeutet, ist dies vorzugsweise Methylenoxy.

Der Ring A ist vorzugsweise trans-1,4-Cyclohexylen, 1,4-Phenylen oder 1,3-Dioxan-2,5-diyl, insbesondere die erst- oder zweitgenannte Gruppe, was auch unabhängig für den Ring C zutrifft. Der Ring B ist seinerseits 2,3-Difluor-1,4-phenylen. Vorzugsweise bedeuten $Z^1$ und $Z^2$ unabhängig voneinander eine einfache Bindung oder eine der Gruppen $Z^1$ und $Z^2$ auch Aethylen. $R^1$ als Alkyl, Alkoxyalkyl oder Alkoxy enthält vorzugsweise von 3 bis 5 Kohlenstoffatome, während $R^2$ als Alkyl vorzugsweise $C_{1-3}$-Alkyl ist.

Die Formel I umfasst vorzugsweise die Verbindungen der folgenden allgemeinen Formeln:

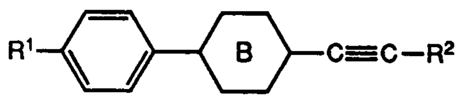 **Ia**

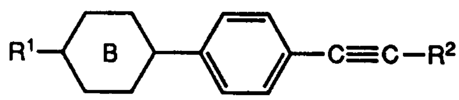 **Ib**

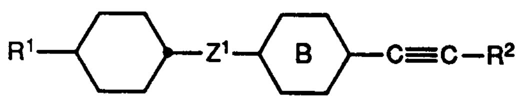 **Ic**

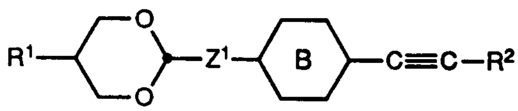 **Id**

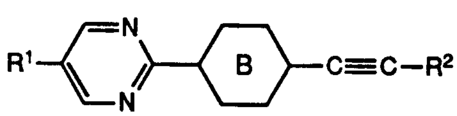 **Ie**

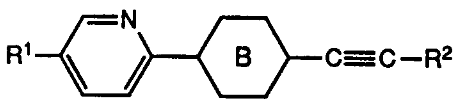 **If**

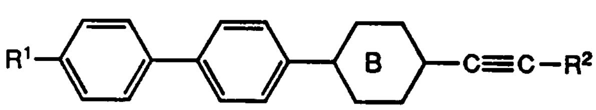 **Ig**

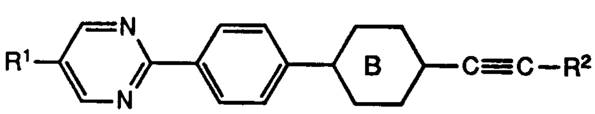 **Ih**

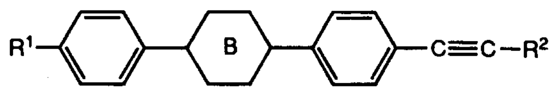 Ii

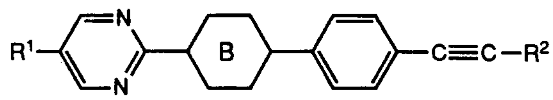 Ij

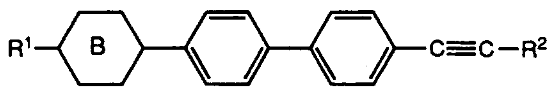 Ik

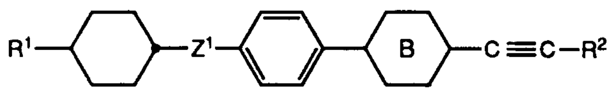 Il

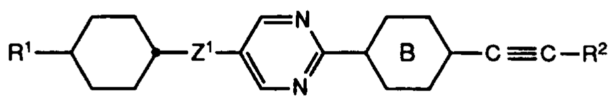 Im

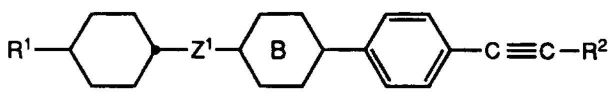 In

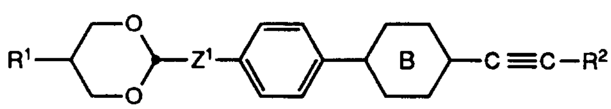 Io

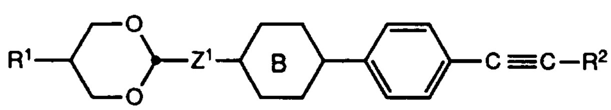 Ip

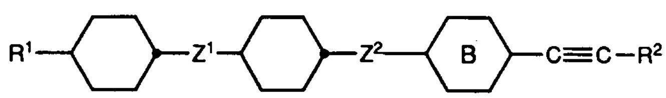 Iq

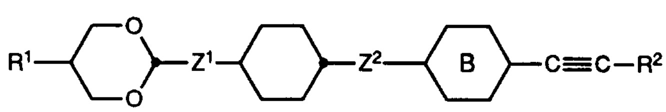 Ir

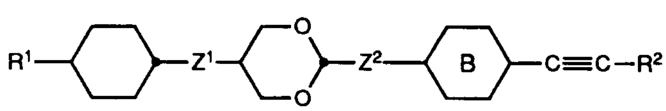 Is

Einzelne Vertreter der Verbindungen der Formel I sind:
1-[4-(4-Propylphenyl)-phenyl]-2,3-difluor-4-(1-propinyl)-benzol,
1-[4-(4-Butylphenyl)-phenyl]-2,3-difluor-4-(1-propinyl)-benzol,
1-[4-(4-Hexylphenyl)-phenyl]-2,3-difluor-4-(1-propinyl)-benzol,

1-[4-(4-Aethylphenyl)-phenyl]-2,3-difluor-4-(1-pentinyl)-benzol,
1-[4-(4-Propylphenyl)-phenyl]-2,3-difluor-4-(1-butinyl)-benzol,
1-[4-(4-Propylphenyl)-phenyl]-2,3-difluor-4-(1-pentinyl)-benzol,
1-[4-(5-Propyl-pyrimidin-2-yl)-phenyl]-2,3-difluor-4-(1-propinyl)-benzol,
1-[4-(5-Pentyl-pyrimidin-2-yl)-phenyl]-2,3-difluor-4-(1-propinyl)-benzol,
1-[4-(5-Propyl-pyridin-2-yl)-phenyl]-2,3-difluor-4-(1-propinyl)-benzol,
1-[4-(5-Pentyl-pyridin-2-yl)-phenyl]-2,3-difluor-4-(1-propinyl)-benzol,
1-[4-(4-trans-Propyl-cyclohexyl)-phenyl]-2,3-difluor-4-(1-propinyl)-benzol,
1-[4-(4-trans-Pentyl-cyclohexyl)-phenyl]-2,3-difluor-4-(1-propinyl)-benzol,
1-[4-trans-(4-trans-Pentyl-cyclohexyl)-cyclohexyl]-2,3-fluor-4-(1-propinyl)-benzol,
1-{4-[2-(4-trans-Propyl-cyclohexyl)-äthyl]-phenyl}-2,3-difluor-4-(1-propinyl)-benzol,
1-{4-trans-[2-(4-trans-Propyl-cyclohexyl)-äthyl]-cyclohexyl}-2,3-difluor-4-(1-propinyl)-benzol,
1-{2-[4-trans-(4-trans-Propylcyclohexyl)-cyclohexyl]-äthyl}-2,3-difluor-4-(1-propinyl)-benzol,
1-(4-Propylphenyl)-2,3-difluor-4-(1-propinyl)-benzol,
1-(4-trans-Propyl-cyclohexyl)-2,3-difluor-4-(1-propinyl)-benzol,
1-(4-trans-Pentyl-cyclohexyl)-2,3-difluor-4-(1-propinyl)-benzol,
1-[2-(4-trans-Propyl-cyclohexyl)-äthyl]-2,3-difluor-4-(1-propinyl)-benzol,
1-(4-Aethylphenyl)-2,3-difluor-4-[4-(1-propinyl)-phenyl]-benzol,
1-(4-Propylphenyl)-2,3-difluor-4-[4-(1-propinyl)-phenyl]-benzol,
1-(4-Butylphenyl)-2,3-difluor-4-[4-(1-propinyl)-phenyl]-benzol,
1-(4-Hexylphenyl)-2,3-difluor-4-[4-(1-propinyl)-phenyl]-benzol,
1-(4-Propylphenyl)-2,3-difluor-4-[4-(1-butinyl)-phenyl]-benzol,
1-(4-Propylphenyl)-2,3-difluor-4-[4-(1-pentinyl)-phenyl]-benzol,
1-(4-Propylphenyl)-2,3-difluor-4-[4-(1-hexinyl)-phenyl]-benzol,
1-(4-Propylphenyl)-2,3-difluor-4-[4-(1-heptinyl)-phenyl]-benzol,
1-(4-trans-Propyl-cyclohexyl)-2,3-difluor-4-[4-(1-propinyl)-phenyl]-benzol,
1-(4-trans-Pentyl-cyclohexyl)-2,3-difluor-4-[4-(1-propinyl)-phenyl]-benzol,
1-[2-(4-trans-Propylcyclohexyl)-äthyl]-2,3-difluor-[4-(1-propinyl)-phenyl]-benzol,
1-(5-Propyl-pyrimidin-2-yl)-2,3-difluor-[4-(1-propinyl)-phenyl]-benzol,
1-(5-Pentylpyrimidin-2-yl)-2,3-difluor-[4-(1-propinyl)-phenyl]-benzol,
1-{4-[4-(1-Propinyl)-phenyl]-phenyl}-2,3-difluor-4-propyl-benzol,
1-{4-[4-(1-Propinyl)-phenyl]-phenyl}-2,3-difluor-4-butyl-benzol,
1-{4-[4-(1-Propinyl)-phenyl]-phenyl}-2,3-difluor-4-hexyl-benzol,
1-{4-[4-(1-Butinyl)-phenyl]-phenyl}-2,3-difluor-4-propyl-benzol,
1-{4-[4-(1-Pentenyl)-phenyl]-phenyl}-2,3-difluor-4-propyl-benzol,
1-{4-[4-trans-(1-Propinyl)-cyclohexyl]-phenyl}-2,3-difluor-4-propyl-benzol,
1-{4-[4-trans-(1-Propinyl)-cyclohexyl]-phenyl}-2,3-difluor-4-pentyl-benzol,
1-[4-{2-[4-trans-(1-Propinyl)-cyclohexyl]-äthyl}-phenyl]-2,3-difluor-4-propyl-benzol,
1-{4-trans-[4-trans-(1-Propinyl)-cyclohexyl]-cyclohexyl}-2,3-difluor-4-propyl-benzol,
1-[4-trans-{2-[4-trans-(1-Propinyl)-cyclohexyl]-äthyl}-cyclohexyl]-2,3-difluor-4-propyl-benzol,
1-[4-(1-Propinyl)-phenyl]-2,3-difluor-4-propyl-benzol,
1-[4-(1-Pentinyl)-phenyl]-2,3-difluor-4-propyl-benzol,
1-{4-[5-(1-Propinyl)-pyrimidin-2-yl]-phenyl}-2,3-difluor-4-propyl-benzol,

und

1-{5-[4-(1-Propinyl)-phenyl]-pyridin-2-yl}-2,3-difluor-4-propyl-benzol.

Die Verbindungen der Formel I können nach an sich bekannten Methoden hergestellt werden, z.B. gemäss dem nachfolgenden Reaktionsschema 1. Die diesbezüglichen Zwischenprodukte bzw. Ausgangsmaterialien sind bekannt oder können nach an sich bekannten Methoden hergestellt werden. Die angewendeten Methoden, die in diesen Reaktionsschemata dargestellt sind, sind reichlich in der Fachliteratur beschrieben und sind dem Fachmann geläufig.

Reaktionsschema 1

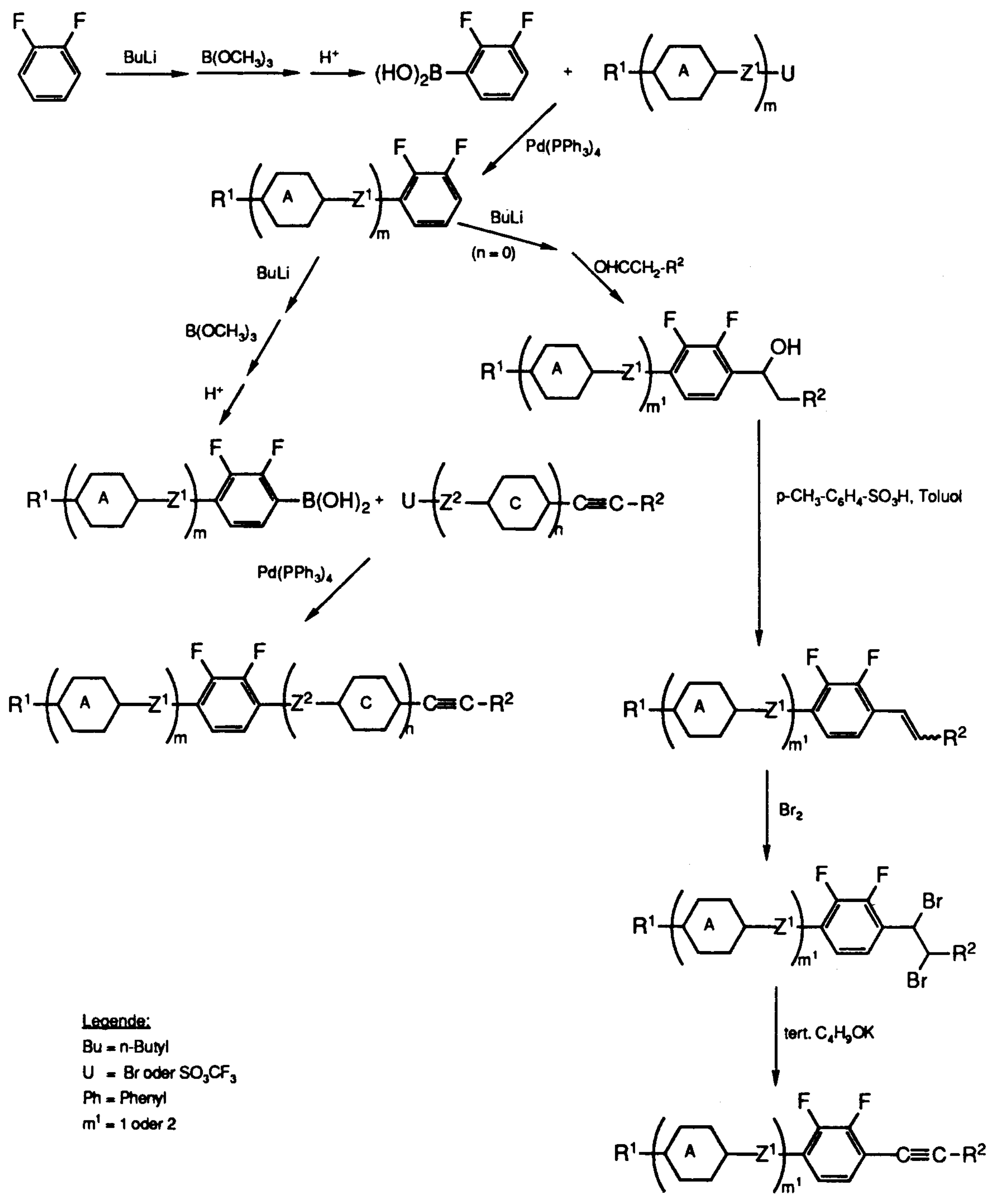


Die Verbindungen der Formel I können in Form von Gemischen untereinander und/oder mit anderen Flüssigkristallkomponenten verwendet werden, wie z.B. mit Substanzen aus den Klassen der Azobenzole, Azoxybenzole, Phenylbenzoate, Cyclohexancarbonsäure-phenylester, Cyclohexancarbonsäure-cyclohexylester, Biphenyle, Phenylcyclohexane, Cyclohexylcyclohexane, Phenylpyrimidine, Cyclohexylpyrimidine, Phenyldioxane, 2-Cyclohexyl-1-phenyläthane, Terphenyle, Cyclohexylbiphenyle, Phenylbicyclohexane und Cyclohexylphenylpyrimidine. Derartige Substanzen sind dem Fachmann bekannt, und viele davon sind zudem im Handel erhältlich.

Die erfindungsgemässen flüssigkristallinen Gemische enthalten mindestens zwei Komponenten, wovon mindestens eine Komponente eine Verbindung der Formel I ist. Eine zweite und gegebenenfalls weitere Komponenten können weitere Verbindungen der Formel I und/oder andersartige Flüssigkristallkomponenten sein.

Die Verbindungen der Formel I eignen sich insbesondere für nematische oder, sofern mindestens eine Komponente des Gemisches optisch aktiv ist, auch für cholesterische Gemische. Ein bevorzugter Anwendungsbereich betrifft die Verwendung als Dielektrikum in Flüssigkristallanzeigevorrichtungen mit verdrillt nematischer Flüssigkristallstruktur, wie TN-Zellen, STN-Zellen, SBE-Zellen, DAP-Zellen, Gast/Wirt-Zellen und OMI-Zellen. Bevorzugte Gemische sind daher diejenigen, welche eine oder mehrere Verbindungen der Formel I und eine oder mehrere Verbindungen mit positiver und/oder negativer dielektrischer Anisotropie enthalten.

Aufgrund der guten Löslichkeit der Verbindungen der Formel I in anderen Flüssigkristallmaterialien und aufgrund ihrer guten Mischbarkeit untereinander kann der Anteil der Verbindungen der Formel I in den erfindungsgemässen Gemischen relativ hoch sein. Im allgemeinen ist ein Anteil von etwa 2-50 Gew.- %, insbesondere etwa 3-30 Gew.-%, an Verbindungen der Formel I bevorzugt.

Vorzugsweise enthalten die erfindungsgemässen Gemische neben einer oder mehreren Verbindungen der Formel I eine oder mehrere Verbindungen aus der Gruppe der Verbindungen der allgemeinen Formeln:

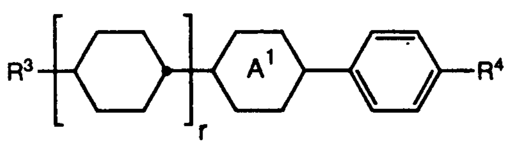

II

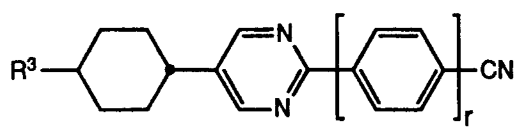

III

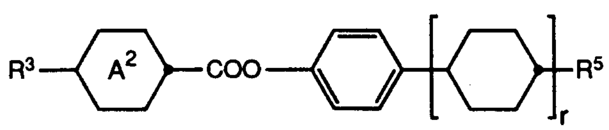

IV

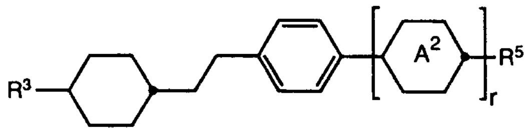
V

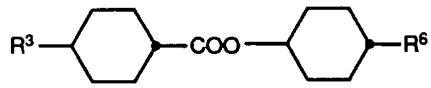
VI

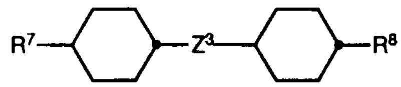
VII

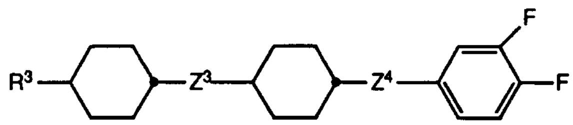
VIII

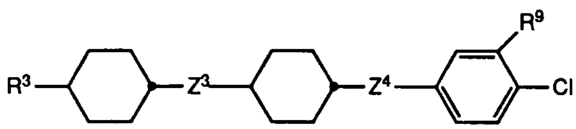
IX

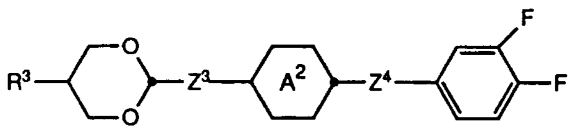
X

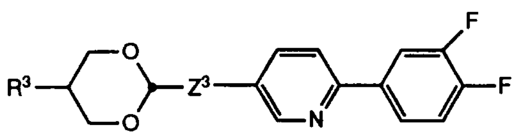
XI

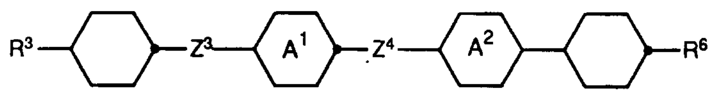
XII

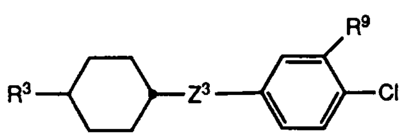
XIII

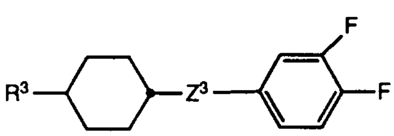
XIV

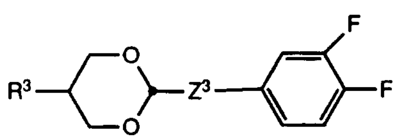
XV

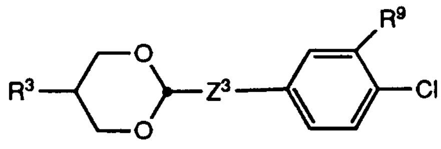

XVI

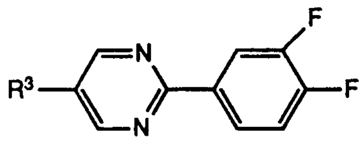

XVII

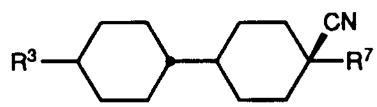

XVIII

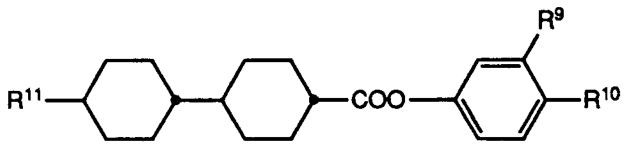

XIX

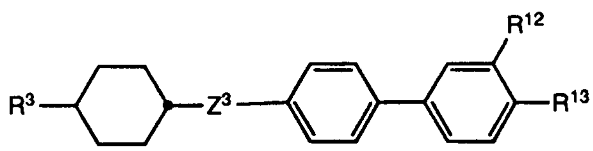

XX

worin r für die Zahl 0 oder 1 steht; $R^3$ und $R^6$ unabhängig voneinander Alkyl, Alkoxyalkyl, 3E-Alkenyl, 4-Alkenyl oder an einem gesättigten Ring auch 1E-Alkenyl bedeutet; Ring $A^1$ 1,4-Phenylen, trans-1,4-Cyclohexylen, Pyridin-2,5-diyl, Pyrimidin-2,5-diyl oder trans-1,3-Dioxan-2,5-diyl darstellt; $R^4$ Cyano, Isothiocyanato, Fluor, Alkyl, 3E-Alkenyl, 4-Alkenyl, Alkoxy, 2E-Alkenyloxy, 3-Alkenyloxy, Difluormethoxy, Trifluormethoxy oder 1-Alkinyl bezeichnet; Ring $A^2$ 1,4-Phenylen oder trans-1,4-Cyclohexylen darstellt; $R^5$ Alkyl, 3E-Alkenyl, 4-Alkenyl oder an einem Cyclohexanring auch 1E-Alkenyl oder an einem Benzolring auch Cyano, Isothiocyanato, Alkoxy, 2E-Alkenyloxy oder 3-Alkenyloxy bedeutet; $R^7$ Alkyl, 1E-Alkenyl, 3E-Alkenyl oder 4-Alkenyl bezeichnet; $Z^3$ und $Z^4$ jeweils eine einfache Bindung oder Aethylen darstellt, wobei zwei aromatische Ringe nur durch eine einfache Bindung gebunden sind; $R^8$ Cyano, Alkyl, 1E-Alkenyl, 3E-Alkenyl, 4-Alkenyl, Alkoxy, 2E-Alkenyloxy, 3-Alkenyloxy, Alkoxymethyl oder (2E-Alkenyl)oxymethyl bedeutet; $R^9$ Wasserstoff, Fluor oder Chlor bezeichnet; $R^{10}$ Fluor, Chlor, Difluormethoxy, Trifluormethoxy oder Cyano bedeutet; $R^{11}$ Alkyl, 1E-Alkenyl, 3E-Alkenyl oder 4-Alkenyl darstellt; $R^{12}$ Wasserstoff oder Fluor bedeutet; und $R^{13}$ Fluor, Chlor, Difluormethoxy oder Trifluormethoxy bezeichnet.

Der obige Ausdruck "gesättigter Ring" umfasst trans-1,4-Cyclohexylen und trans-1,3-Dioxan-2,5-diyl. Reste $R^3$ bis $R^8$ und $R^{11}$ besitzen vorzugsweise höchstens je 12 Kohlenstoffatome, besonders bevorzugt höchstens je 7 Kohlenstoffatome. Geradkettige Reste sind im allgemeinen bevorzugt.

Die Herstellung der flüssigkristallinen Gemische und der elektro-optischen Vorrichtungen kann in an sich bekannter Weise erfolgen.

Die Erfindung wird durch die folgenden Beispiele weiter veranschaulicht. In den Beispielen 1-3 bedeuten Klp. Klärpunkt, C eine kristalline Phase, $S_B$ eine smektische B-Phase, N eine nematische Phase und I die isotrope Phase.

Beispiel 1

a) Zu einer Lösung von 3,75g 4-Pentyl-biphenyl-4'-trifluormethylsulfonat in 30ml Dimethoxyäthan werden unter Rühren 1,74g 2,3-Difluorbenzolboransäure und 0,15g Tetrakis-(triphenylphosphin)palladium (0) und anschliessend 15ml 2M Natriumcarbonatlösung zugefügt. Dann wird 15 Stunden auf Rückflusstemperatur gehalten, anschliessend abgekühlt, zwischen Wasser und Diäthyläther verteilt und die organische Phase über wasserfreiem Magnesiumsulfat getrocknet. Nach Abdampfen des Lösungsmittels wird der Rückstand an Kieselgel mit 3%-igem Essigester

in Petroläther chromatographiert. Ausbeute: 3,15g an farblosem, festem 1-[4-(4-Pentylphenyl)-phenyl]-2,3-difluorbenzol.

b) Zu einer Lösung von 3,15g 1-[4-(4-Pentylphenyl)-phenyl]-2,3-difluorbenzol in 35ml trockenem Tetrahydrofuran werden bei -50°C innert 5 Minuten 6,45ml 1,6N Butyllithiumlösung in n-Hexan getropft und das Reaktionsgemisch bei gleicher Temperatur 3 Stunden reagieren gelassen. Dann wird eine Lösung von 652 mg Propionaldehyd in 4ml Tetrahydrofuran zugefügt und das Gemisch langsam auf Raumtemperatur erwärmen gelassen. Nach 15 Stunden wird mit 10ml 1N Salzsäure versetzt, zwischen Methylenchlorid und Wasser verteilt, die organische Phase mit gesättigter Natriumbicarbonatlösung gewaschen und über wasserfreiem Magnesiumsulfat getrocknet. Abdampfen des Lösungsmittels und Chromatographie des Rückstandes an Kieselgel mit 5-20%-igem Essigester in Petroläther ergibt 3,2g 1-[4-(4-Pentylphenyl)-phenyl]-2,3-difluor-4-(1-hydroxypropyl)-benzol.

c) Eine Lösung von 3,2g 1-[4-(4-Pentylphenyl)-phenyl]-2,3-difluor-4-(1-hydroxypropyl)-benzol und 0,3g p-Toluolsulfonsäure in 70ml Toluol wird eine Stunde bei Rückflusstemperatur erhitzt, dann abgekühlt, mit gesättigter Natriumbicarbonatlösung gewaschen und über wasserfreiem Magnesiumsulfat getrocknet. Eindampfen des Lösungsmittels ergibt 3,03g 1-[4-(4-Pentylphenyl)-phenyl]-2,3-difluor-4-(1-propenyl)-benzol als rohes Z/E-Isomerengemisch.

d) Zu einer Lösung von 3,03g rohem 1-[4-(4-Pentylphenyl)-phenyl]-2,3-difluor-4-(1-propenyl)-benzol in 60ml Chloroform wird bei 0°C langsam eine Lösung von 1,41g Brom in 25ml Chloroform getropft. Nach 30 Minuten werden 30ml 10%-ige Natriumbisulfitlösung zugefügt, die organische Phase abgetrennt, mit Wasser mehrmals gewaschen, über wasserfreiem Magnesiumsulfat getrocknet und eingedampft. Als Rückstand bleiben 4,3g 1-[4-(4-Pentylphenyl)-phenyl]-2,3-difluor-4-(1,2-dibrompropyl)-benzol.

e) Zu einer Lösung von 4,3g rohem 1-[4-(4-Pentylphenyl)-phenyl]-2,3-difluor-4-(1,2-dibrompropyl)benzol in 200ml tert.Butylmethyläther werden 2,5g Kalium-tert.butylat gegeben, und das Reaktionsgemisch wird 4,5 Stunden bei Rückflusstemperatur erhitzt. Danach wird abgekühlt, mit 50ml Wasser versetzt und zwischen Wasser und Diäthyläther verteilt. Die vereinten organischen Phasen werden über wasserfreiem Magnesiumsulfat getrocknet, das Lösungsmittel abgedampft und der Rückstand an Kieselgel mit 5%-igem Essigester in Petroläther chromatographiert. Filtration über Alox (CAMAG, neutral, Aktivierungsstufe 1) und fraktionierte Kristallisation aus Essigester ergibt 2,04g 1-[4-(4-Pentylphenyl)-phenyl]-2,3-difluor-4-(1-propinyl)-benzol, Smp. (C-$S_B$) 135,7°C; $S_B$-N 173,6°C; Klp. (N-I) ca. 210°C.

Auf analoge Weise werden folgende Verbindungen I hergestellt:

1-[4-trans-(4-trans-Propyl-cyclohexyl)-cyclohexyl]-2,3-difluor-4-(1-propinyl)-benzol, Smp. 99,9°C; Klp. (N-I) 240°C.

1-(4-Pentylphenyl)-2,3-difluor-4-(1-propinyl)-benzol, Smp. 45,5°C.

Beispiel 2

a) Zu einer Lösung von 1-Brom-4-pentyl-benzol in 40ml Toluol wird zunächst 40ml 2 M Natriumcarbonatlösung sowie 0,71g Tetrakis (triphenylphosphin)palladium (0) und danach eine Lösung von 4g 2,3-Difluorbenzolboronsäure in 20ml Aethanol gegeben, und das Reaktionsgemisch wird bei Rückflusstemperatur gehalten. Dann wird abgekühlt, zwischen Wasser und Diäthyläther verteilt, die organische Phase über wasserfreiem Magnesiumsulfat getrocknet und die organische Phase eingedampft. Chromatographie an Kieselgel mit Petroläther ergibt 3,49g 1-(4-Pentylphenyl)-2,3-difluor-benzol.

b) Zu einer Lösung von 1,75g 1-(4-Pentylphenyl)-2,3-difluor-benzol in 15ml trockenem Tetrahydrofuran wird bei -70°C eine Lösung von 4,6 ml Buthyllithium (1,6N) in n-Hexan innert 5 Minuten getropft. Nach 3 Stunden bei -70°C wird bei derselben Temperatur eine Lösung von 0,838g Trimethylborat in 10 ml trockenem Tetrahydrofuran innert 15 Minuten zugetropft, dann langsam auf Raumtemperatur erwärmen gelassen und 15 Stunden bei dieser Temperatur gehalten. Anschliessend werden 5 ml 1N Salzsäure zugefügt, ca. 30 Minuten gerührt und dann zwischen Diäthyläther und Wasser verteilt. Die organische Phase wird hierauf mit 1N Natriumhydroxydlösung extrahiert, dann die wässrige Phase mit konzentrierter Salzsäure sauer gestellt, mit Methylenchlorid extrahiert und über wasserfreiem Magnesiumsulfat getrocknet. Nach Filtration und Abdampfen des Lösungsmittels bleiben 1,78g 4-(4-Pentylphenyl)-2,3-difluor-benzolboronsäure als ölige Masse.

c) Eine Lösung von 1g 1-(1-Propinyl)-4-brom-benzol (hergestellt aus 4-Brombenzaldehyd durch Wittigreaktion, Bromierung des resultierenden Aethans und Bromelimination analog Beispiel 1e) wird mit 700mg Tetrakis (triphenylphosphin)palladium (0) und 1,53g 4-(4-Pentylphenyl)-2,3-difluorbenzolboronsäure wie unter a) beschrieben umgesetzt. Chromatographie an Kieselgel mit 3%-igem Essigester in Petroläther und fraktionierte Kristallisation aus Essigester ergibt 0,84g 1-(4-Pentylphenyl)-2,3-difluor-4-[4-(1-propinyl)-phenyl]-benzol, Smp. 102,9°C; Klp. (N-I) 198,9°C.

Beispiel 3

a) Zu einer Lösung von 15g 4-(4-Bromphenyl)-propiophenon (hergestellt durch Acylierung von p-Brombiphenyl) in 150ml Methylenchlorid und 25ml 2,6-Di-tert-butylpyridin wird bei Raumtemperatur eine Lösung von 10,2ml Trifluormethansulfonsäure in 10ml Methylenchlorid getropft und 15 Stunden gerührt. Dann wird vorsichtig auf eine gesättigte Natriumbicarbonatlösung getropft, die organische Phase abgetrennt, gewaschen, über wasserfreiem Magnesiumsulfat getrocknet und eingedampft. Der Rückstand (49,5g) wird mit Petroläther digeriert, auf -20° C abgekühlt und genutscht. Chromatographie des Festkörpers (15,7g) an Kieselgel mit 10%-igem Essigester in Petroläther und anschliessende Filtration über Alox (CAMAG, neutral, Aktivierungsstufe 1) ergibt 9,76g reines 1-(4-Bromphenyl)-4-(1-propinyl)-benzol.

b) Umsatz von 0,915g 1-(4-Bromphenyl)-4-(1-propinyl)-benzol mit 1g 1-2,3-Difluor-4-pentyl-benzolboransäure analog zu Beispiel 2c ergibt 0,7g 1-{4-[4-(1-Propinyl)-phenyl]-phenyl}-2,3-difluor-4-pentyl-benzol, Smp. 127°C; Klp. (N-I) 216°C.

Auf analoge Weise wird folgende Verbindung I hergestellt:
1-[4-(1-Propinyl)-phenyl]-2,3-difluor-4-pentyl-benzol, Smp. 52,5°C

Beispiel 4

Zur Untersuchung der Eigenschaften der Verbindungen der Formel I in Gemischen (M) wurden mehrere binäre Gemische (BM) hergestellt, und zwar jeweils aus 4-(trans-4-Pentyl-cyclohexyl)-benzonitril (5CP) und jeweils einer Verbindung der Formel I. Der Einfluss auf den Klärpunkt (Klp.), auf die Schwellenspannung V10 [die Spannung in Volt (V) für 10%-ige Transmission in der Blickrichtung senkrecht zur Plattenoberflächel, auf die Ansprechzeiten t in Millisekunden (Einschaltzeit $t_{on}$ bzw. Auschaltzeit $t_{off}$) sowie auf die optische Anisotropie $\Delta n$ wurden untersucht. $V_{10}$, $t_{on}$ und $t_{off}$ wurden in einer TN-Zelle (low bias tilt) mit 8µm Plattenabstand bei 22°C gemessen; als Betriebsspannung wurde der 2,5-fache Wert der Schwellenspannung $V_{10}$ gewählt.

Die verwendeten Komponenten der Formel I, deren Konzentration im jeweiligen binären Gemisch und die gemessenen Daten sind in der nachfolgenden Tabelle zusammengefasst. Zum Vergleich sind die entsprechenden Werte für reines 4-(trans-4-Pentyl-cyclohexyl)-benzonitril ebenfalls angegeben. Schliesslich sind auch noch die berechneten $\alpha$-Werte aufgeführt, wobei

$$\alpha = \frac{Klp.(5CP) \cdot t_{off}(M)}{Klp.(M) \cdot t_{off}(5CP)}.$$

Tabelle

| Binäre Gemische mit 4-(trans-4-Pentyl-cyclohexyl)-benzonitril | | | | | | |
|---|---|---|---|---|---|---|
| Komponent der Formel I, Konzentration im BM in Gew.-% | Klp.(°C) | $V_{10}$(V) | $t_{on}$(ms) bei 22°C | $t_{off}$(ms) bei 22°C | $\Delta n$ | $\alpha$ |
| [100% 4-(trans-4-Pentylcyclohexyl)-benzonitril] | 54,6 | 1,64 | 22 | 40 | 0,120 | 1,00 |
| 1-(4-Pentylphenyl)-2,3-difluor-4-(1-propinyl)-benzol, 10% | 49,0 | 1,56 | 26 | 44 | 0,129 | 1,23 |
| 1-(4-Pentylphenyl)-2,3-difluor-4-(1-propinyl)-benzol, 20% | 44,1 | 1,39 | 31 | 55 | 0,135 | 1,70 |
| 1-(4-Pentylphenyl)-2,3-difluor-4-[4-(1-propinyl)-phenyl]-benzol, 10% | 62,6 | 1,74 | 27 | 46 | 0,132 | 1,00 |
| 1-(4-Pentylphenyl)-2,3-difluor-4-[4-(1-propinyl)-phenyl]-benzol, 20% | 72,6 | 1,90 | 29 | 48 | 0,162 | 0,90 |
| 1-{4-[4-(1-Propinyl)-phenyl]-phenyl}-2,3-difluor-4-pentyl-benzol, 10% | 60,1 | 1,66 | 27 | 43 | 0,137 | 0,98 |
| 1-{4-[4-(1-Propinyl)-phenyl]-phenyl}-2,3-difluor-4-pentyl-benzol, 20% | 74,1 | 1,86 | 30 | 48 | 0,163 | 0,88 |
| 1-[4-(1-Propinyl)-phenyl]-2,3-difluor-4-pentyl-benzol, 10% | 49,6 | 1,62 | 27 | 44 | 0,129 | 1,21 |
| 1-[4-(1-Propinyl)-phenyl]-2,3-difluor-4-pentyl-benzol, 20% | 44,6 | 1,55 | 29 | 49 | 0,135 | 1,50 |

**Patentansprüche**

1. Verbindungen der allgemeinen Formel

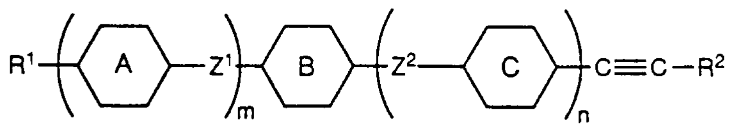

I

worin

die Ringe A und C unabhängig voneinander trans-1,4-Cyclohexylen, 1-4-Phenylen, trans-1,3-Dioxan-2,5-diyl, Pyridin-2,5-diyl oder Pyrimidin-2,5-diyl sind, mit der Massgabe, dass höchstens einer der Ringe A und C Pyridin-2,5-diyl oder Pyrimidin-2,5-diyl sein kann,

der Ring B 2,3-difluorsubstituiertes 1,4-Phenylen ist,

$Z^1$ oder $Z^2$ eine einfache Bindung, Aethylen, Oxymethylen oder Methylenoxy bedeuten,

m und n unabhängig voneinander 0, 1 oder 2 bedeuten, wobei die Summe m + n 1 oder 2 beträgt,

$R^1$ $C_{1-10}$-Alkyl, $C_{2-9}$-Alkoxyalkyl oder $C_{1-9}$-Alkoxy bedeutet, und

$R^2$ $C_{1-10}$-Alkyl bedeutet,

mit der Massgabe, dass zwei 1,4-Phenylenringe nur durch eine einfache Bindung miteinander verknüpft sein können.

2. Verbindungen nach Anspruch 1, dadurch gekennzeichnet, dass der Ring A trans-1,4-Cyclohexylen oder 1,4-Phenylen ist.

3. Verbindungen nach einem der Ansprüche 1 bis 2, dadurch gekennzeichnet, dass der Ring C trans-1,4-Cyclohexylen oder 1,4-Phenylen ist.

4. Verbindungen nach einem der Ansprüche 1 bis 3, dadurch gekennzeichnet, dass $Z^1$ eine einfache Bindung oder Aethylen bedeutet.

5. Verbindungen nach einem der Ansprüche 1 bis 4, dadurch gekennzeichnet, dass $Z^2$ eine einfache Bindung oder Aethylen bedeutet.

6. Verbindungen nach einem der Ansprüche 1 bis 5, dadurch gekennzeichnet, dass $R^1$ 3 bis 5 Kohlenstoffatome enthält.

7. Verbindungen nach einem der Ansprüche 1 bis 6, dadurch gekennzeichnet, dass $R^2$ $C_{1-3}$-Alkyl bedeutet.

8. Flüssigkristallines Gemisch mit mindestens 2 Komponenten, dadurch gekennzeichnet, dass mindestens eine Komponente eine Verbindung der in Anspruch 1 definierten Formel I ist.

9. Flüssigkristallines Gemisch nach Anspruch 8, dadurch gekennzeichnet, dass der Anteil an Verbindungen der Formel I 3-30 Gew.-% beträgt.

10. Flüssigkristallines Gemisch nach Anspruch 8 oder 9, dadurch gekennzeichnet, dass es eine oder mehrere Verbindungen der Formel I und eine oder mehrere Verbindungen aus der Gruppe der Verbindungen der allgemeinen Formeln

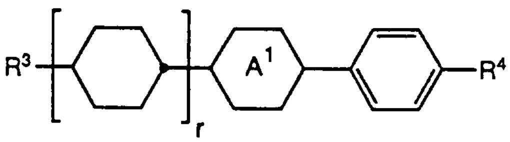

II

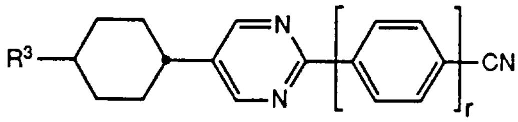

III

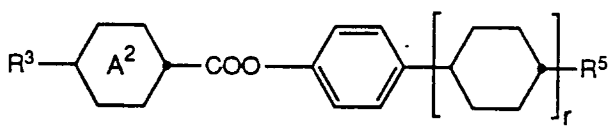

IV

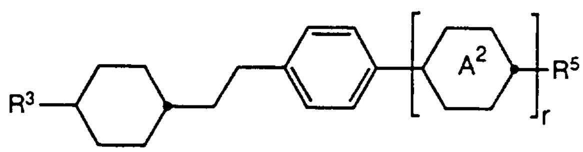

V

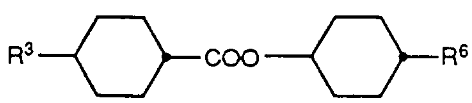

VI

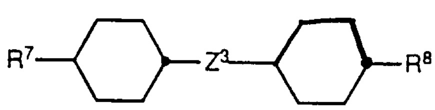

VII

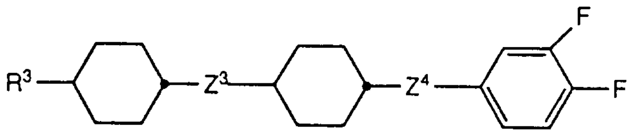

VIII

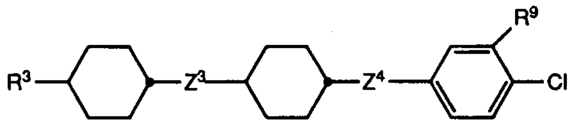

IX

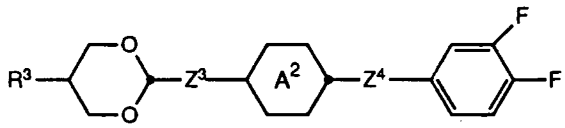

X

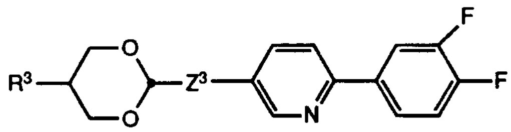

XI

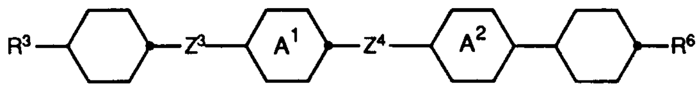

XII

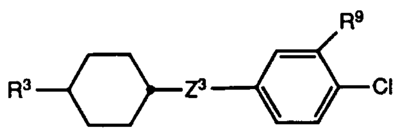

XIII

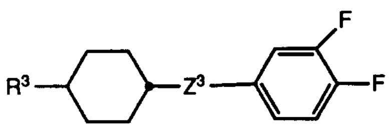

XIV

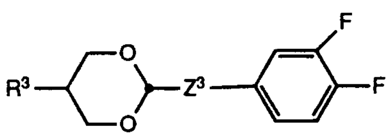

XV

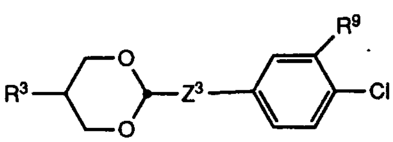

XVI

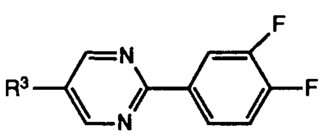

XVII

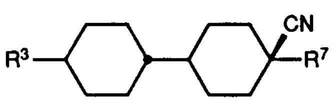

XVIII

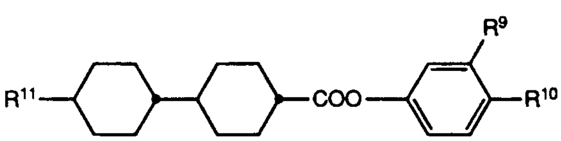

XIX

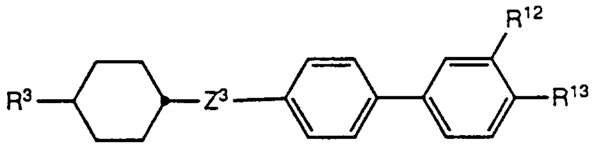

XX

worin r für die Zahl 0 oder 1 steht; $R^3$ und $R^6$ unabhängig voneinander Alkyl, Alkoxyalkyl, 3E-Alkenyl, 4-Alkenyl oder an einem gesättigten Ring auch 1E-Alkenyl bedeutet; Ring $A^1$ 1,4-Phenylen, trans-1,4-Cyclohexylen, Pyridin-2,5-diyl, Pyrimidin-2,5-diyl oder trans-1,3-Dioxan-2,5-diyl darstellt; $R^4$ Cyano, Isothiocyanato, Fluor, Alkyl, 3E-Alkenyl, 4-Alkenyl, Alkoxy, 2E-Alkenyloxy, 3-Alkenyloxy, Difluormethoxy, Trifluormethoxy oder 1-Alkinyl bezeichnet; Ring $A^2$ 1,4-Phenylen oder trans-1,4-Cyclohexylen darstellt; $R^5$ Alkyl, 3E-Alkenyl, 4-Alkenyl oder an einem Cyclohexanring auch 1E-Alkenyl oder an einem Benzolring auch Cyano, Isothiocyanato, Alkoxy, 2E-Alkenyloxy oder 3-Alkenyloxy bedeutet; $R^7$ Alkyl, 1E-Alkenyl, 3E-Alkenyl oder 4-Alkenyl bezeichnet; $Z^3$ und $Z^4$ jeweils eine einfache Bindung oder Aethylen darstellt, wobei zwei aromatische Ringe nur durch eine einfache Bindung gebunden sind; $R^8$ Cyano, Alkyl, 1E-Alkenyl, 3E-Alkenyl, 4-Alkenyl, Alkoxy, 2E-Alkenyloxy, 3-Alkenyloxy, Alkoxymethyl oder (2E-Alkenyl)oxymethyl bedeutet; $R^9$ Wasserstoff, Fluor oder Chlor bezeichnet; $R^{10}$ Fluor, Chlor, Difluormethoxy, Trifluormethoxy oder Cyano bedeutet; $R^{11}$ Alkyl, 1E-Alkenyl, 3E-Alkenyl oder 4-Alkenyl darstellt; $R^{12}$ Wasserstoff oder Fluor bedeutet; und $R^{13}$ Fluor, Chlor, Difluormethoxy oder Trifluormethoxy bezeichnet,
enthält.

**11.** Verwendung der Verbindungen der in Anspruch 1 definierten Formel I für elektro-optische Zwecke.

## Claims

**1.** Compounds of the general formula

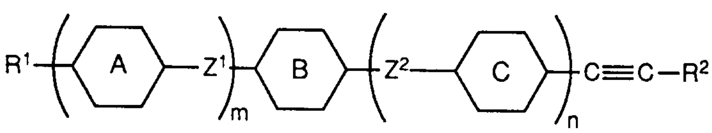

I

wherein

rings A and C each individually can be trans-1,4-cyclohexylene, 1,4-phenylene, trans-1,3-dioxane-2,5-diyl, pyridine-2,5-diyl or pyrimidine-2,5-diyl, with the proviso that a maximum of one of rings A and C can be pyridine-2,5-diyl or pyrimidine-2,5-diyl,

ring B is 2,3-difluoro-substituted 1,4-phenylene,

$Z^1$ or $Z^2$ signifies a single bond and $Z^2$ or $Z^1$, respectively, signifies a single bond, ethylene, oxymethylene or methyleneoxy,

m and n each independently signify 0, 1 or 2, with the sum m + n being 1 or 2,

$R^1$ signifies $C_{1-10}$-alkyl, $C_{2-9}$-alkoxyalkyl or $C_{1-9}$-alkoxy and

$R^2$ signifies $C_{1-10}$-alkyl,

with the proviso that two 1,4-phenylene rings can be linked with each other only by a single bond.

**2.** Compounds according to claim 1, characterized in that ring A is trans-1,4-cyclohexylene or 1,4-phenylene.

**3.** Compounds according to any one of claims 1 to 2, characterized in that ring C is trans-1,4-cyclohexylene or 1,4-phenylene.

**4.** Compounds according to any one of claims 1 to 3, characterized in that $Z^1$ signifies a single bond or ethylene.

**5.** Compounds according to any one of claims 1 to 4, characterized in that $Z^2$ signifies a single bond or ethylene.

**6.** Compounds according to any one of claims 1 to 5, characterized in that $R^1$ contains 3 to 5 carbon atoms.

7. Compounds according to any one of claims 1 to 6, characterized in that $R^2$ signifies $C_{1-3}$-alkyl.

8. A liquid crystalline mixture with at least 2 components, characterized in that at least one component is a compound of formula I defined in claim 1.

9. A liquid crystalline mixture according to claim 8, characterized in that the content of compounds of formula I is 3-30 wt.%.

10. A liquid crystalline mixture according to claim 8 or 9, characterized in that it contains one or more compounds of formula I and one or more compounds from the group of compounds of the general formulae

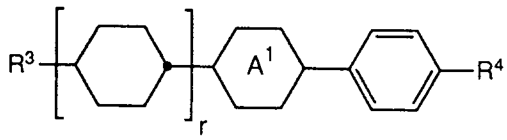

II

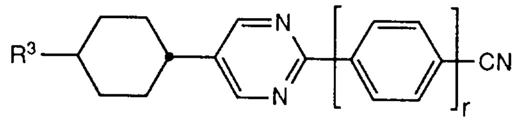

III

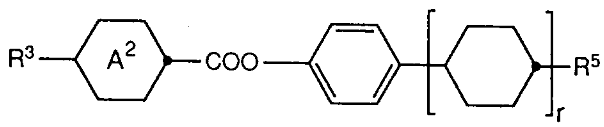

IV

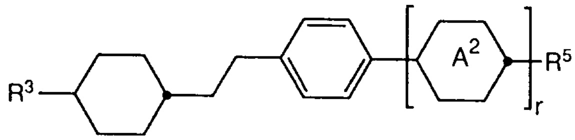

V

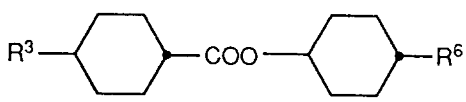

VI

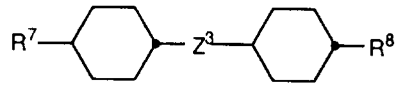
VII

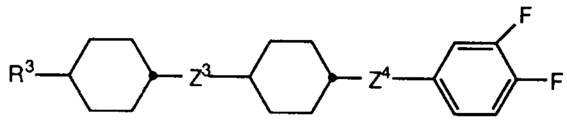
VIII

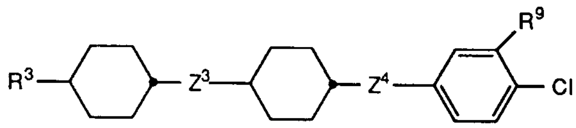
IX

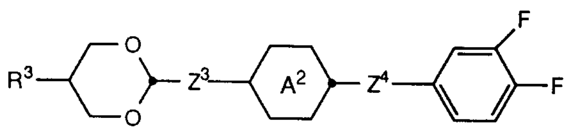
X

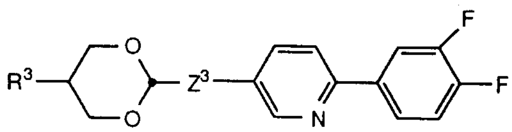
XI

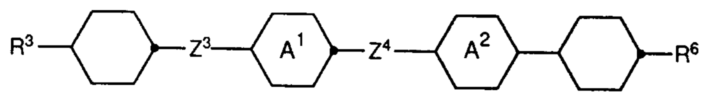
XII

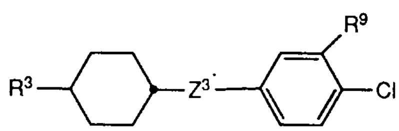
XIII

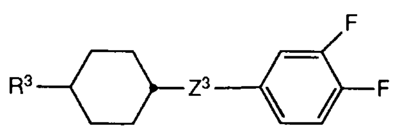
XIV

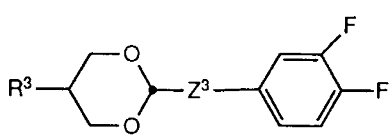
XV

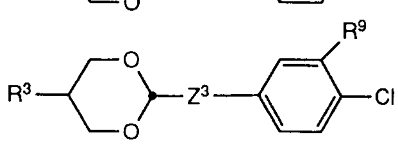
XVI

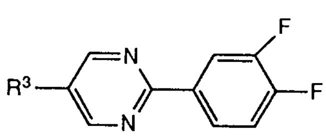
XVII

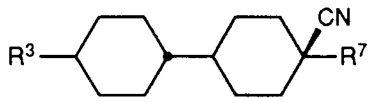

XVIII

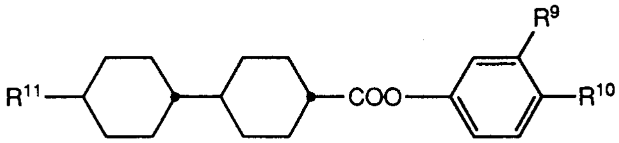

XIX

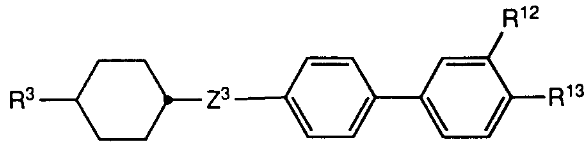

XX

wherein r stands for the number 0 or 1; $R^3$ and $R^6$ each independently signify alkyl, alkoxyalkyl, 3E-alkenyl, 4-alkenyl or on a saturated ring also 1E-alkenyl; ring $A^1$ represents 1,4-phenylene, trans-1,4-cyclohexylene, pyridine-2,5-diyl, pyrimidine-2,5-diyl or trans-1,3-dioxane-2,5-diyl; $R^4$ denotes cyano, isothiocyanato, fluorine, alkyl, 3E-alkenyl, 4-alkenyl, alkoxy, 2E-alkenyloxy, 3-alkenyloxy, difluoromethoxy, trifluoromethoxy or 1-alkynyl; ring $A^2$ represents 1,4-phenylene or trans-1,4-cyclohexylene; $R^5$ signifies alkyl, 3E-alkenyl, 4-alkenyl or on a cyclohexane ring also 1E-alkenyl or on a benzene ring also cyano, isothiocyanato, alkoxy, 2E-alkenyloxy or 3-alkenyloxy; $R^7$ denotes alkyl, 1E-alkenyl, 3E-alkenyl or 4-alkenyl; $Z^3$ and $Z^4$ each represent a single bond or ethylene, whereby two aromatic rings are linked only by a single bond; $R^8$ signifies cyano, alkyl, 1E-alkenyl, 3E-alkenyl, 4-alkenyl, alkoxy, 2E-alkenyloxy, 3-alkenyloxy, alkoxymethyl or (2E-alkenyl)oxymethyl; $R^9$ denotes hydrogen, fluorine or chlorine; $R^{10}$ signifies fluorine, chlorine, difluoromethoxy, trifluoromethoxy or cyano; $R^{11}$ represents alkyl, 1E-alkenyl, 3E-alkenyl or 4-alkenyl; $R^{12}$ signifies hydrogen or fluorine; and $R^{13}$ denotes fluorine, chlorine, difluoromethoxy or trifluoromethoxy.

**11.** The use of the compounds of formula I defined in claim 1 for electro-optical purposes.

## Revendications

**1.** Composés de formule générale

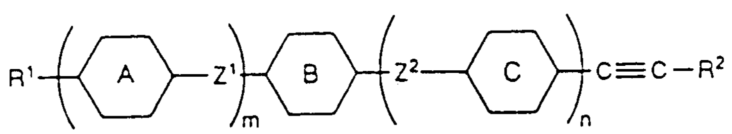

I

dans laquelle
les cycles A et C sont indépendamment les uns des autres, des groupements trans-1,4-cyclohexylène, 1,4-phénylène, trans-1,3-dioxanne-2,5-diyle, pyridine-2,5-diyle ou pyrimidine-2,5-diyle, avec la condition qu'au plus un des cycles A et C peut être le groupement pyridine-2,5-diyle ou pyrimidine-2,5-diyle,
le cycle B est le groupement 1,4-phénylène difluorosubstitué en 2,3,
$Z^1$ ou $Z^2$ signifient une liaison simple, un groupement éthylène, oxyméthylène ou méthylénoxy,
m et n signifient indépendamment les uns des autres 0, 1 ou 2, où la somme m + n vaut 1 ou 2,
$R^1$ signifie un groupement alkyle en $C^1$ à $C^{10}$, alcoxyalkyle en $C^2$ à $C^9$ ou alcoxy en $C^1$ à $C^9$,
$R^2$ signifie un groupement alkyle en $C^1$ à $C^{10}$,
avec la condition que deux cycles 1,4-phénylène ne peuvent être liés que par une liaison simple.

**2.** Composés selon la revendication 1, caractérisés en ce que le cycle A est le groupement trans-1,4-cyclohexylène ou 1,4-phénylène.

3. Composés selon l'une quelconque des revendications 1 à 2, caractérisés en ce que le cycle C est le groupement trans-1,4-cyclohexylène ou 1,4-phénylène.

4. Composés selon l'une quelconque des revendications 1 à 3, caractérisés en ce que $Z^1$ signifie une liaison simple ou un groupement éthylène.

5. Composés selon l'une quelconque des revendications 1 à 4, caractérisés en ce que $Z^2$ signifie une liaison simple ou groupement éthylène.

6. Composés selon l'une quelconque des revendications 1 à 5, caractérisés en ce que $R^1$ renferme 3 à 5 atomes de carbone.

7. Composés selon l'une quelconque des revendications 1 à 6, caractérisés en ce que $R^2$ signifie un groupement alkyle en $C^1$ à $C^3$.

8. Mélange cristaux liquides avec au moins 2 composants, caractérisé en ce qu'au moins un composant est un composé de la formule I définie dans la revendication 1.

9. Mélange cristaux liquides selon la revendication 8, caractérisé en ce que la part de composés de formule I s'élève de 3 à 30% en poids.

10. Mélange cristaux liquides selon la revendication 8 ou 9, caractérisé en ce qu'il contient un ou plusieurs composés de formule I et un ou plusieurs composés parmi le groupe des composés de formules générales:

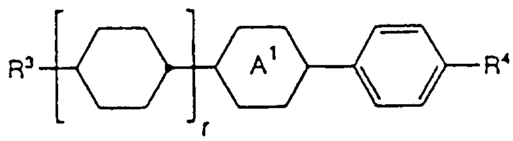 II

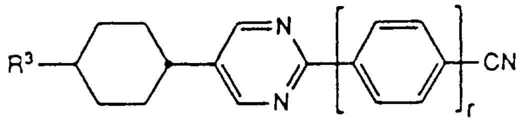 III

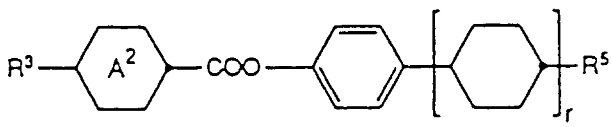 IV

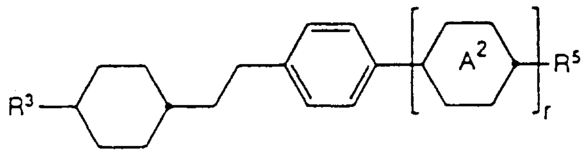 V

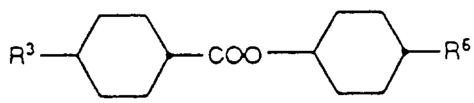 VI

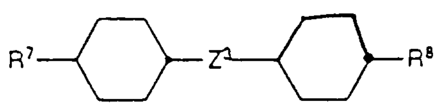 VII

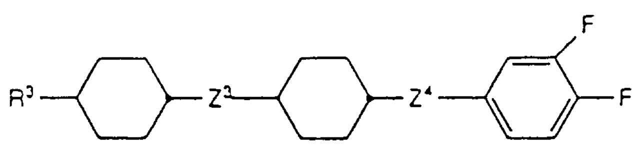 VIII

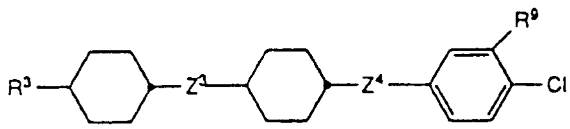

IX

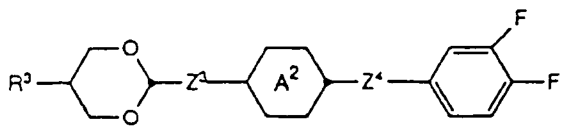

X

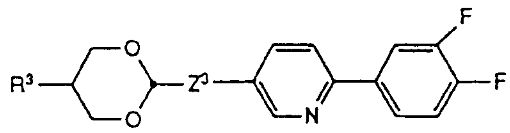

XI

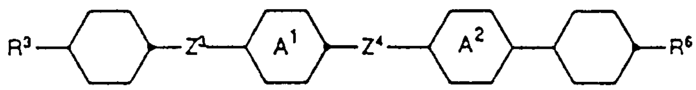

XII

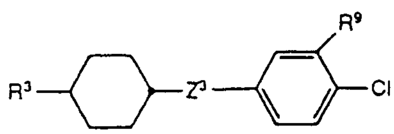

XIII

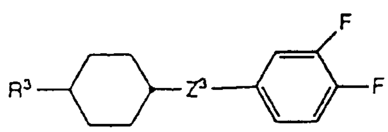

XIV

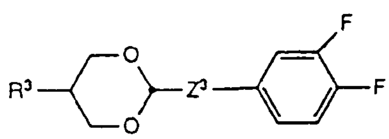

XV

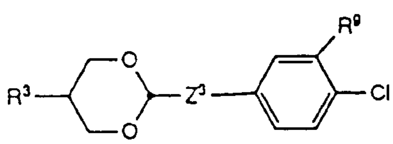

XVI

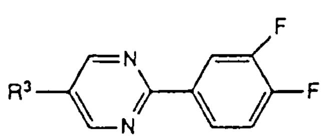

XVII

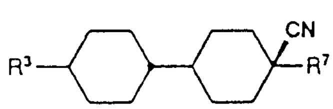

XVIII

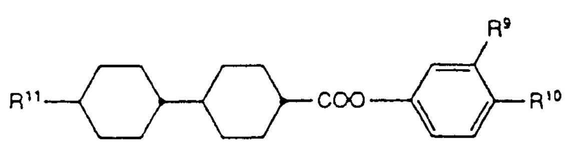

XIX

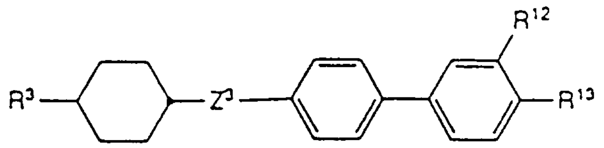

XX

dans lesquelles r représente le noire 0 ou 1; $R^3$ et $R^6$ signifient indépendamment les uns des autres un groupement alkyle, alcoxyalkyle, 3E-alcényle, 4-alcényle ou également 1E-alcényle sur un cycle saturé; le cycle $A^1$ représente le groupement 1,4-phénylène, trans-1,4-cyclohexylène, pyridine-2,5-diyle ou trans-1,3-dioxanne-2,5-diyle; $R^4$ désigne un groupement cyano, isothiocyanato, un atome de fluor, un groupement alkyle, 3E-alcényle, 4-alcényle, alcoxy, 2E-alcényloxy, 3-alcényloxy, difluorométhoxy, trifluorométhoxy ou 1-alcynyle; le cycle $A^2$ représente le groupement 1,4-phénylène ou trans-1,4-cyclohexylène; $R^5$ désigne un groupement alkyle, 3E-alcényle, 4-alcényle, ou également 1E-alcényle sur un cycle cyclohexane ou également un groupement cyano, isothiocyano, alcoxy, 2E-alcényloxy, 3-alcényloxy sur un noyau benzénique; $R^7$ désigne un groupement alkyle, 1E-alcényle, 3E-alcényle ou 4-alcényle; $Z^3$ et $Z^4$ représente chacun une liaison simple ou un groupement éthylène, dans lesquelles deux cycles aromatiques ne sont liés que par une liaison simple; $R^8$ signifie un groupement cyano, alkyle, 1E-alcényle, 3E-alcényle, 4-alcényle, alcoxy, 2E-alcényloxy, 3-alcényloxy, alcoxyméthyle ou (2E-alcényl)oxyméthyle; $R^9$ signifie un atome d'hydrogène, de fluor ou de chlore; $R^{10}$ signifie un atome de fluor, de chlore, un groupement difluorométhoxy, trifluorométhoxy ou cyano; $R^{11}$ représente un groupement alkyle, 1E-alcényle, 3E-alcényle, 4-alcényle; $R^{12}$ signifie un atome d'hydrogène ou de fluor; et $R^{13}$ signifie un atome de fluor, de chlore, un groupement difluorométhoxy ou trifluorométhoxy.

**11.** Utilisation des composés de la formule I définie dans la revendication 1 à des fins électrooptiques.